# FASCICULE DE BREVET EUROPEEN

(11) **EP 1 395 234 B1**
(45) Date de publication et mention de la délivrance du brevet: **28.07.2010**
(21) Numéro de dépôt: 02738272.0
(22) Date de dépôt: 17.05.2002
(51) Int. Cl.: A61K 8/73, A61Q 5/00

(54) **COMPOSITIONS COSMETIQUES CONTENANT UN AMIDON ET UN ESTER ET LEURS UTILISATIONS**
KOSMETISCHE ZUSAMMENSETZUNGEN MIT EINER STÄRKE UND EINEM ESTER UND DEREN VERWENDUNG
COSMETIC COMPOSITIONS CONTAINING A STARCH AND AN ESTER AND THE USE THEREOF

(30) Priorité: 18.05.2001 FR 0106603
(43) Date de publication de la demande: 10.03.2004
(73) Titulaire: L'Oréal, 75008 Paris (FR)
(72) Inventeur: MAGNET, Armelle, F-69840 Irigny (FR); DECOSTER, Sandrine, F-95210 Saint Gratien (FR); CHESNEAU, Laurent, F-92300 Levallois Perret (FR)
(74) Mandataire: Le Blainvaux Bellegarde, Françoise
(86) Numéro de dépôt international: PCT/FR2002/001686
(87) Numéro de publication internationale: WO 2002/094207

(56) Documents cités:
- EP-A- 0 418 443
- EP-A- 1 093 807
- EP-A1- 0 797 979
- EP-A2- 0 562 638
- WO-A-96/22073
- WO-A-99/48472
- WO-A1-89/04652
- AU-B2- 716 510
- FR-A- 2 774 286
- FR-A- 2 775 897
- FR-A- 2 801 500
- FR-A1- 2 320 729
- FR-A1- 2 348 263
- US-A- 5 665 292
- US-A- 5 683 706

## Description

La présente invention concerne de nouvelles compositions cosmétiques comprenant dans un milieu cosmétiquement acceptable au moins un ester d'acide carboxylique particulier et au moins un amidon.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératiniques et en particulier des cheveux, des compositions cosmétiques contenant des polysaccharides épaississants tels que notamment l'amidon ou les celluloses.

De telles compositions présentent cependant des inconvénients tels que des problèmes de rinçabilité, des problèmes de stabilité à pH acide, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques pour faciliter le démêlage des cheveux et pour leur communiquer douceur et souplesse. L'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

On a déjà proposé d'utiliser en tant qu'agent conditionneur des huiles telles que les huiles végétales ou animales ou encore des esters d'acides gras. Cependant, les matières kératiniques traitées avec ces compositions présentent le plus souvent un toucher gras rédhibitoire.

En résumé, il s'avère que les compositions cosmétiques actuelles ne donnent pas complètement satisfaction.

WO 96/22073 décrit des compositions cosmétiques contenant des amidons non réticulés pour épaissir et stabiliser les émulsions. Ce document ne décrit pas les esters gras selon l'invention.

WO89 04652 divulgue une composition comprenant un amidon et un ester gras mais ne contenant pas de tensioactif cationique.

AU 716 510 et EP 797 979 décrivent des compositions comprenant un amidon mais ne contenant pas d'esters gras.

EP 562 638 décrit de nombreux esters gras comme additifs de compositions capillaires.

La demanderesse a maintenant découvert que l'association d'un amidon avec des esters carboxyliques particuliers permet de remédier à ces inconvénients.

Ainsi, à la suite d'importantes recherches menées sur la question, il a maintenant été trouvé par la Demanderesse que l'utilisation de compositions en particulier capillaires à base d'esters particuliers et d'amidon, permettait de limiter, voire supprimer, les problèmes évoqués ci-dessus. Les cheveux se démêlent facilement et sont lisses de la racine à la pointe, la tenue de la coiffure est améliorée.

De plus, cette association apporte une texture fondante aux compositions cosmétiques, c'est à dire qui disparaît rapidement dans la chevelure. Les cheveux traités avec cette composition ont un toucher doux et sans résidus.

Par ailleurs, les compositions de invention appliquées sur la peau notamment sous forme de bain moussant ou de gel douche, apportent une amélioration de la douceur de la peau.

Ainsi, selon la présente invention, il est maintenant proposé de nouvelles compositions cosmétiques capillaires, comprenant, dans un milieu aqueux cosmétiquement acceptable,
a- au moins un amidon choisi parmi les phosphates de mono amidon, les phosphates de diamidon, les phosphates de triamidon et leurs mélanges, et
b- au moins un ester carboxylique choisi parmi :
les esters liquides d'acide carboxylique en C3-C30 et d'alcool en C1-C30, l'un au moins de l'acide ou de l'alcool étant ramifié, et
   c- au moins un agent tensioactif cationique,
sous réserve que :
ladite composition comprenne une concentration en savons d'acide gras inférieure ou égale à 1,5% en poids par rapport au poids total de la composition,
la composition comprend au moins 30% en poids d'eau et moins de 20% en poids de phase grasse,
la composition comprend moins de 10% en poids d'esters gras,
la composition ne comprend pas de polymère cationique non siliconé lorsque l'amidon est un phosphate d'amidon,
lorsque la composition comprend un dérivé phosphorylé d'amidon, au moins un agent épaississant et moins de 3% en poids de tensioactif, l'ester n'est pas le palmitate d'isopropyle ou l'adipate de diisopropyle.

Un autre objet de l'invention concerne l'utilisation d'amidon dans, ou pour la fabrication d'une composition cosmétique comprenant un ester tel que défini ci-dessus.

Un autre objet de l'invention concerne l'utilisation pour le traitement des cheveux d'une composition cosmétique telle que définie ci-dessus.

Un autre objet de l'invention concerne l'utilisation pour le conditionnement des cheveux d'une composition cosmétique telle que définie ci-dessus.

Les différents objets de l'invention vont maintenant être détaillés. L'ensemble des significations et définitions des composés utilisés dans la présente invention données ci-dessous sont valables pour l'ensemble des objets de l'invention.

Par savons d'acides gras, on comprend selon l'invention un sel d'un métal alcalin ou alcalinoterreux ou d'une amine grasse et d'un acide gras en C10-C18, notamment l'acide stéarique et l'acide isostéarique.

Par exempte de savons d'acides gras, on comprend selon l'invention que la concentration en savons d'acide gras est inférieure ou égale à environ 1,5% en poids, de préférence inférieure à environ 0,5% en poids et plus particulièrement inférieure à 0,1% en poids par rapport au poids total de la composition.

La phase grasse comprend tous les corps gras insoluble dans l'eau à température ambiante de la composition tels que notamment les esters gras, les huiles végétales, minérales, synthétiques, les alcools gras, les acides gras, les cires, les silicones. La phase grasse représente de préférence de 0,001 à 15% en poids et plus particulièrement de 0,01 à 10% en poids par rapport au poids total de la composition et encore plus particulièrement de 0,5 à 8% en poids.

La composition selon l'invention comprend moins de 10% en poids d'esters gras. Par esters gras, on comprend les esters selon invention ainsi que les esters d'acide carboxylique en C3-C30 et d'alcool en C1-C30 n'ayant pas l'un au moins de l'acide ou de l'alcool ramifié ou possédant au moins une double-liaison carbone-carbone.

Les amidons utilisables dans la présente invention sont plus particulièrement des macromolécules sous forme de polymères constitués de motifs élémentaires qui sont des unités anhydroglucose. Le nombre de ces motifs et leur assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine botanique des amidons.

Les molécules d'amidons utilisés dans la présente invention peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

Les amidons se présentent généralement sous la forme d'une poudre blanche, insoluble dans l'eau froide, dont la taille des particules élémentaires va de 3 à 100 microns.

Les amidons utilisés dans la composition de l'invention peuvent être modifiés par une ou plusieurs des réactions suivantes : prégélatinisation, oxydation, réticulation, estérification, traitements thermiques.

De manière plus particulière, ces réactions peuvent être réalisées de la façon suivante :
- prégélatinisation en faisant éclater les granules d'amidon (par exemple séchage et cuisson dans un tambour sécheur) ;
- oxydation par des oxydants forts conduisant à l'introduction de groupes carboxyle dans la molécule d'amidon et à la dépolymèrisation de la molécule d'amidon (par exemple en traitant une solution aqueuse d'amidon par l'hypochlorite de sodium) ;
- réticulation par des agents fonctionnels capables de réagir avec les groupes hydroxyle des molécules d'amidon qui vont ainsi être liées entre elles (par exemple avec des groupes glyceryl et/ou phosphate) ;
- estérification en milieu alcalin pour le greffage de groupes fonctionnels, notamment acyl en en C1-C6 (acétyl), hydroxyalkylés en C1-C6 (hydroxyéthyl, hydroxypropyl), carboxyméthyl, octénylsuccinique.

On peut notamment obtenir par réticulation avec des composés phophorés des phosphates de monoamidon (du type Am-O-PO-(OX)₂), des phosphates de diamidon ( du type Am-O-PO-(OX)-O-Am) ou même de triamidon (du type Am-O-PO- (O-Am)₂) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'omithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE ou STRUCTURE ZEA de NATIONAL STARCH (phosphate de diamidon de maïs gélatinisé).

Selon l'invention, on peut aussi utiliser des amidons amphotères, ces amidons amphotères contiennent un ou plusieurs groupements anioniques et un ou plusieurs groupements cationiques. Les groupements anioniques et cationiques peuvent être liés au même site réactif de la molécule d'amidon ou à des sites réactifs différents; de préférence ils sont liés au même site réactif. Les groupements anioniques peuvent être de type carboxylique, phosphate ou sulfate et de préférence carboxylique. Les groupements cationiques peuvent être de type amine primaire, secondaire, tertiaire ou quaternaire.

Les amidons amphotères sont notamment choisis parmi les composés de formules suivantes : 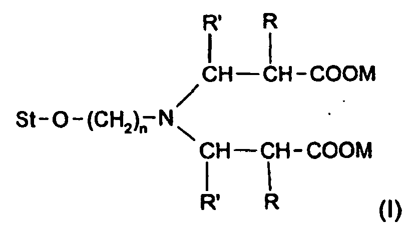 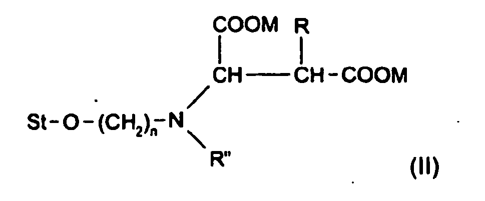 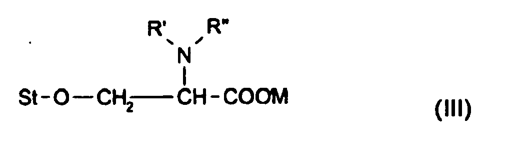 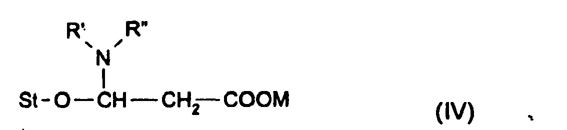 formules dans lesquelles :
St-O représente une molécule d'amidon,
R, identique ou différent, représente un atome d'hydrogène ou un radical méthyle,
R', identique ou différent, représente un atome d'hydrogène, un radical méthyle ou un groupement -COOH,
n est un entier égal à 2 ou 3,
M, identique ou différent, désigne un atome d'hydrogène, un métal alcalin ou alcalinoterreux tels que Na, K, U, NH₄ , un ammonium quaternaire ou une amine organique,
R" représente un atome d'hydrogène ou un radical alkyle ayant de 1 à 18 atomes de carbone.

Ces composés sont notamment décrits dans les brevets US 5,455,340 et US 4,017,460.

Les molécules d'amidons peuvent être issues de toutes les sources végétales d'amidon telles que notamment le maïs, la pomme de terre, l'avoine, le riz, le tapioca, le sorgho, l'orge ou le blé. On peut également utiliser les hydrolysats des amidons cités ci-dessus. L'amidon est de préférence issu de la pomme de terre.

On utilise particulièrement les amidons de formules (I) ou (II). On utilise plus particulièrement les amidons modifiés par de l'acide 2-chloroéthyl aminodipropionique, c'est à dire les amidons de formule (I) ou (II) dans lesquelles R, R', R" et M représentent un atome d'hydrogène et n est égal à 2.

Le ou les amidons sont utilisés de préférence en une quantité allant de 0,01 à 20% en poids par rapport au poids total de la composition. Plus préférentiellement, cette quantité va de 0,05 à 15% en poids par rapport au poids total de la composition et encore plus préférentiellement de 0,1 à 10% en poids.

De préférence les esters selon l'invention sont des esters liquides insolubles dans l'eau. Les esters d'acides carboxyliques liquides insolubles dans l'eau sont insolubles dans l'eau à une concentration supérieure ou égale à 0,1% en poids dans l'eau à 25°C, c'est à dire qu'ils ne forment pas, dans ces conditions, une solution macroscopiquement isotrope transparente.

Le nombre total de carbone des esters de l'invention est généralement supérieur ou égal à 10 et de préférence inférieure à 100 et plus particulièrement inférieur à 80.

Selon l'invention, les esters utilisés sont liquides à température ambiante (de 20 à 30°C).

Les esters liquides d'acide carboxylique en C3-C30 et d'alcool en C1-C30, l'un au moins de l'acide ou de l'alcool étant ramifié ou insaturé, sont notamment choisis parmi les esters d'acide carboxylique en C6-C24 et d'alcool en C3-C20.

On peut citer le béhénate d'octyldodécyle ; le béhénate d'isocétyle ; le lactate d'isocétyle ; le lactate d'isostéaryle ; le lactate de linoléyle ; le lactate d'oléyle ; l'octanoate de isostéaryle ; l'octanoate d'isocétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle ; l'isostéarate de myristyle ; l'isononanoate d'octyle ; l'isononate de 2-éthylhexyle; l'isostéarate d'octyle ; l'érucate d'octyldodécyle ; les palmitates d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, l'isostéarate de butyle, le stéarate d'isobutyle ; le laurate de 2-hexyldécyle.

On peut également utiliser les esters d'acides di ou tricarboxyliques en C4-C22 et d'alcools en C1-C22 et les esters d'acides mono di ou tricarboxyliques et d'alcools di, tri, tétra ou pentahydroxy en C2-C26.

On peut notamment citer : le sébacate de diisopropyle ; l'adipate de diisopropyle ; l'adipate de diisostéaryle ; le stéarate d'octyldodécyl stéaroyl; le tétraisononanoate de pentaérythrityle ; le tétraisostéarate de pentaérythrityle ; le citrate de triisopropyle ; le citrate de trisotéaryle; le citrate de trioctyldodécyle.

De préférence, l'acide et l'alcool de l'ester sont saturés.

Parmi les esters cités ci-dessus, on préfère utiliser le palmitate d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, le isostéarate de butyle, le stéarate d'isobutyle ; le laurate de 2-hexyldécyle et l'isononanate d'isononyle.

Selon l'invention, le ou les esters d'acide carboxylique peuvent représenter de 0,001 % à moins de 10 % en poids, de préférence de 0,01 % à 8 % en poids, et encore plus préférentiellement de 0,05 % à 6 % en poids, du poids total de la composition finale.

Le rapport en poids ester de type 1)/ amidon est de préférence inférieur ou égal à 1,5 et plus particulièrement inférieur à 1. Lorsque la composition contient un ester de type 2), le rapport ester /amidon est de préférence inférieur à 2,5 et plus particulièrement inférieur à 2.

Selon un mode de réalisation particulièrement préféré, les compositions selon l'invention comprennent en outre au moins un agent bénéfique pour les cheveux tels que notamment les silicones, les huiles végétales, animales, minérales ou de synthèse, les cires, les céramides, les pseudocéramides, les polymères cationiques, les filtres solaires, les vitamines.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition compris entre 60° C et 260° C, et plus particulièrement encore parmi:
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/ méthylakylsiloxane, tel que la "SILICONE VOLATILE FZ 3109" commercialisée par la société UNION CARBIDE, de structure chimique : 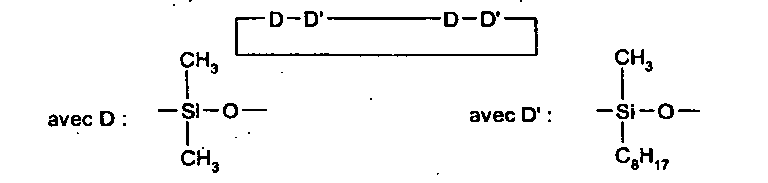 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
. les huiles SILBIONE de la série 70 641 de RHONE POULENC ;
. les huiles des séries RHODORSIL 70 633 et 763 de RHONE POULENC ;
. l'huile DOW CORNING 556 COSMETIC GRAD FLUID de DOW CORNING ;
. les silicones de la série PK de BAYER comme le produit PK20 ;
. les silicones des séries PN, PH de BAYER comme les produits PN1000 et PH1000 ;
. certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinyisiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :
- les mélanges formés à partir d'un polydiméthylsiloxane hydroxylé en bout de chaîne (dénommé diméthiconol selon la nomenclature du dictionnaire CTFA) et d'un polydiméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (IX) : 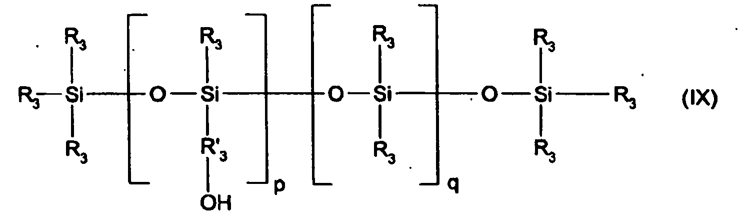 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p est compris entre 1 et 30 inclus ; q est compris entre 1 et 150 inclus ;
- des groupements acyloxyalkyle tels que par exemple les polyorganosiloxanes décrits dans le brevet US-A-4957732 et répondant à la formule (X) : 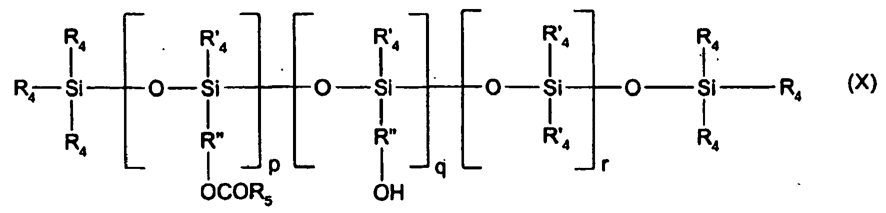 dans laquelle :
   R₄ désigne un groupement méthyle, phényle, -OCOR₅, hydroxyle, un seul des radicaux R₄ par atome de silicium pouvant être OH ;
   R'₄ désigne méthyle, phényle ; au moins 60 % en proportion molaire de l'ensemble des radicaux R₄ et R'₄ désignant méthyle ;
   R₅ désigne alkyle ou alcényle en C₈-C₂₀;
   R" désigne un radical alkylène hydrocarboné divalent, linéaire ou ramifié en C₂-C₁₈ ;
   r est compris entre 1 et 120 inclus ;
   p est compris entre 1 et 30 ;
   q est égal à 0 ou est inférieur à 0,5 p, p + q étant compris entre 1 et 30 ; les polyorganosiloxanes de formule (VI) peuvent contenir des groupements : 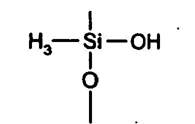
   dans des proportions ne dépassant pas 15 % de la somme p + q + r.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule: 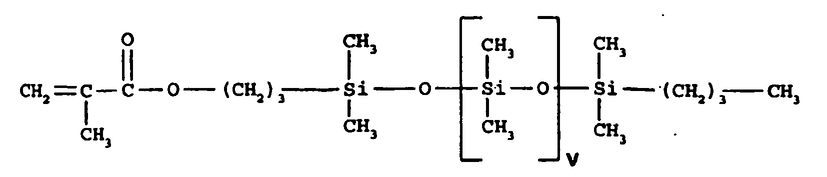
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, on peut également utiliser des polyuréthanes siliconés, notamment ceux décrits dans les demandes de brevet EP 0 751 162, EP 0 619 111.

Selon l'invention, toutes les silicones peuvent également être utilisées sous forme d'émulsions, de nanoémulsions ou de micrémulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société CALGON, les polymères quaternaires de vinylpyrrolidone et de vinylimidazole, les homopolymères ou copolymères réticulés de sels de méthacryloyloxyalkyl(C₁-C₄) trialkyl(C₁-C₄)ammonium et leurs mélanges.

Selon l'invention, les agents bénéfiques additionnels peuvent représenter de 0,001 % à 20 % en poids, de préférence de 0,01 % à 10% en poids et plus particulièrement de 0,1 à 5% en poids par rapport au poids total de la composition finale.

Les compositions de l'invention contiennent en outre avantageusement au moins un agent tensioactif qui est généralement présent en une quantité comprise entre 0,01% et 50% en poids environ, de préférence entre 0,1% et 40% et encore plus préférentiellement entre 0,5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants:

### (i) Tensioactif(s) anionique(s):

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHONE POULENC.

### (iv) Les tensioactifs cationiques peuvent être choisis parmi :

A) les sels d'ammonium quaternaires de la formule générale (IV) suivante : 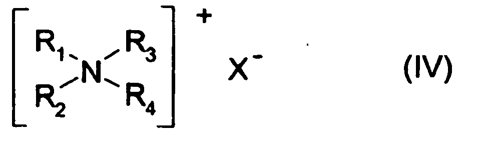 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   , et
   a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de béhényl triméthyl ammonium.
   b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone; R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) - les sels d'ammonium quaternaire de l'imidazolinium, comme par exemple celui de formule (V) suivante : 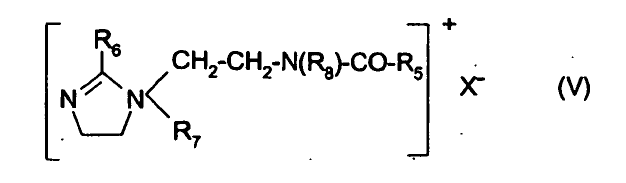 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quatemium-27(CTFA 1997) ou le Quaternium-83 (CTFA 1997) commercialisés sous les dénominations "REWOQUAT" W 75, W90, W75PG, W75HPG par la société WITCO,
C) - les sels de diammonium quaternaire de formule (VI) : 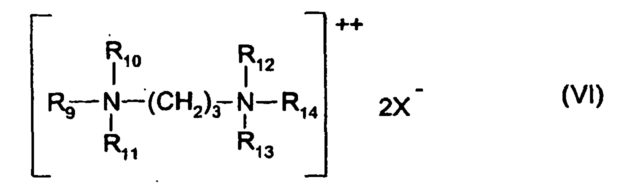 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) - les sels d'ammonium quaternaire contenant au moins une fonction ester de formule (VII) suivante : 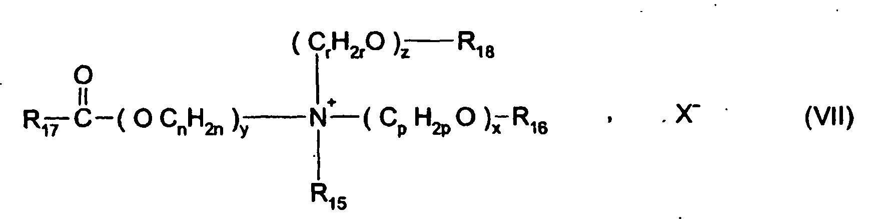 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
   - le radical 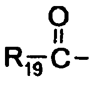
   - les radicaux R₂₀ hydrocarbonés en C₁-C₂₂ linéaires ou ramifiés, saturés ou insaturés,
   - l'atome d'hydrogène,
   - R₁₈ est choisi parmi :
   - le radical 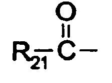
   - les radicaux R₂₂ hydrocarbonés en C₁-C₆ linéaires ou ramifiés, saturés ou insaturés,
   - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X- est un anion simple ou complexe, organique ou inorganique ;
      sous réserve que la somme x + y + z vaut de 1 à 15 , que lorsque x vaut 0 alors R₁₆ désigne R₂₀ et que lorsque z vaut 0 alors R₁₈ désigne R₂₂.
      On utilise plus particulièrement les sels d'ammonium de formule (VII) dans laquelle
   - R₁₅ désigne un radical méthyle ou éthyle,
   - x et y sont égaux à 1 ;
   - z est égal à 0 ou 1 ;
   - n, p et r sont égaux à 2 ;
   - R₁₆ est choisi parmi :
   - le radical 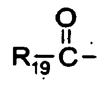
   - les radicaux méthyle, éthyle ou hydrocarbonés en C₁₄-C₂₂
   - l'atome d'hydrogène ;
   - R₁₇, R₁₉ et R₂₁, identiques ou différents, sont choisis parmi les radicaux hydrocarbonés en C₇-C₂₁, linéaires ou ramifiés, saturés ou insaturés ;
   - R₁₈ est choisi parmi :
   - le radical 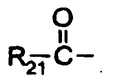
   - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quatemium-27 ou le Quatemium-83 commercialisés par la société WITCO.

La composition comprend de préférence de 30 à 99% d'eau, en particulier de 50 à 95% d'eau et encore plus particulièrement de 70 à 95% en poids d'eau par rapport au poids total de la composition.

Le milieu aqueux cosmétiquement acceptable comprend uniquement de l'eau ou un mélange d'eau et d'un solvant cosmétiquement acceptable choisi parmi les alcools inférieurs en C₁-C₄, tels que l'éthanol, l'isopropanol, le tertio-butanol ou le n-butanol ; les polyols comme le propylèneglycol ; les éthers de polyols ; les alcanes en C₅-C₁₀ ; les cétones en C₃₋₄ comme l'acétone et la méthyléthylcétone ; les acétates d'alkyle en C₁-C₄ comme l'acétate de méthyle, l'acétate d'éthyle et l'acétate de butyle ; le diméthoxyéthane, le diéthoxyéthane ; et leurs mélanges.

Le pH des compositions de l'invention est compris entre 4 et 8, de préférence entre 5 et 7.

La composition de l'invention peut également contenir au moins un additif choisi parmi les épaississants, les parfums, les agents nacrants, les conservateurs, les filtres solaires siliconés ou non, les vitamines, les provitamines, les polymères anioniques ou non ioniques, les protéines, les hydrolysats de protéines, l'acide méthyl-18 eicosanoique, les hydroxyacides, les vitamines, les provitamines telles que le panthénol, et tout autre additif classiquement utilisé dans le domaine cosmétique qui n'affecte pas les propriétés des compositions selon l'invention.

L'homme de métier veillera à choisir les éventuels additifs et leur quantité de manière à ce qu'ils ne nuisent pas aux propriétés des compositions de la présente invention.

Ces additifs sont éventuellement présents dans la composition selon l'invention dans des proportions pouvant aller de 0,001 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions de l'invention peuvent se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Selon un mode de réalisation préféré de l'invention, la composition peut être utilisée comme après-shampoing.

Lorsque la composition se présente sous la forme d'un après-shampooing ou d'une composition de soin éventuellement à rincer, elle contient avantageusement au moins un tensioactif cationique, sa concentration est généralement comprise entre 0,1 et 10% en poids et de préférence de 0,5 à 5% en poids par rapport au poids total de la composition.

Elle peut également contenir un ou plusieurs tensioactifs amphotères ou non ioniques.

Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse.

Le ou les tensioactifs constituant la base lavante peuvent être indifféremment choisis, seuls ou en mélanges, au sein des tensioactif anioniques, amphotères, non ioniques et cationiques tels que définit, ci-dessus. La base lavante contient au moins un tensioactif détergent.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₅-C₁₆) sulfonate de sodium et leurs mélange avec:
soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
   - soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des cheveux, caractérisé en ce qu'il consiste à appliquer sur les cheveux une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique et plus particulièrement les cheveux.

Les compositions selon l'invention peuvent également se présenter sous forme de lotions aqueuses ou hydroalcooliques pour le soin des cheveux.

Les compositions cosmétiques selon l'invention peuvent se présenter sous forme de gel, de lait, de crème, d'émulsion, de lotion épaissie ou de mousse et être utilisées pour les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants.

Dans les exemples, MA signifie matière active.

### EXEMPLE 1

On a réalisé un après-shampooing conforme à l'invention de composition suivante :
- Phosphate de diamidon de maïs prégélatinisé
   (STRUCTURE ZEA de NATIONAL STARCH) 5,3 g MA
- Myristate d'isopropyle 1 g
- Chlorure de béhényl triméthyl ammonium 3,2 g MA
- Alcool cétylstéarylique (50/50 en poids) 4g
- Eau qsp 100 g

La composition a une texture gélifiée et très fondante lors de l'application sur des cheveux humides. Sa rinçabilité est bonne. Les cheveux mouillés ne sont pas chargés et la mise en forme est aisée.

### EXEMPLE 2

On a réalisé un après-shampooing conforme à l'invention de composition suivante :
- Fécule de pomme de terre modifiée par
   l'acide 2-chloroéthyl aminodipropionique
   neutralisée par la soude, (Solanace Starch de
   NATIONAL STARCH) 1,5 g
- Isononanoate d'isononyle 1g
- Quatemium-87 (REWOQUAT WPG 75 de WITCO) 3,75 g MA
- Alcool cétylstéarylique (50/50 en poids) 4g
- Eau qsp 100 g

Les cheveux traités ont les mêmes propriétés que ceux traités avec la composition de l'exemple 1.

### EXEMPLE 3

On a réalisé un après-shampooing conforme à l'invention de composition suivante :
- Fécule de pomme de terre modifiée par
   l'acide 2-chloroéthyl aminodipropionique
   neutralisée par la soude (Solanace Starch de
   NATIONAL STARCH) 3 g
- Benzoate d'alkyle en C12-C15
   (FINSOLV TN de GOLDSCHMIDT) 5 g
- Benzophénone-3 (UVINUL M40 de BASF) 1 g
- Myristate d'isopropyle 0,8 g
- Quatemium-87 (REWOQUAT WPG 75 de WITCO) 3,75 g MA
- Alcool cétylstéarylique (50/50 en poids) 4g
- Eau qsp 100 g

Les cheveux traités ont les mêmes propriétés que ceux traités avec la composition de l'exemple 1.

### EXEMPLE 4

On a réalisé un après-shampooing conforme à l'invention de composition suivante :
- Fécule de pomme de terre modifiée par
   l'acide 2-chloroéthyl aminodipropionique
   neutralisée par la soude, (Solanace Starch de
   NATIONAL STARCH) 3 g
- Myristate d'isopropyle 0,6 g
- Mélange d'alcool cétylstéarylique (50/50 en poids)
   et d'alcool cétylstéarylique oxyéthyléné (30 OE)
   (SINNOWAX AO de COGNIS) 5 g
- Eau qsp 100 g

Les cheveux traités ont les mêmes propriétés que ceux traités avec la composition de l'exemple 1.

## Revendications

1. Composition cosmétique, capillaire **caractérisée par le fait qu'**elle comprend, dans un milieu cosmétiquement acceptable,
a - au moins un amidon choisi parmi les phosphates de mono amidon, les phosphates de diamidon, les phosphates de triamidon et leurs mélanges, et parmi les amidons amphotères.
b - au moins un ester carboxylique choisi parmi:
les esters liquides d'acide carboxylique en C3-C30 et d'alcool en C1-C30, l'un au
moins de l'acide ou de l'alcool étant ramifié, et
c - au moins un agent tensioactif cationique.
sous réserve que :
ladite composition comprenne une concentration en savons d'acide gras inférieure ou
égale à 1,5% en poids par rapport au poids total de la composition,
la composition comprend au moins 30% en poids d'eau et moins de 20% en poids de phase grasse,
la composition comprend moins de 10% en poids d'esters gras,
la composition ne comprend pas de polymère cationique non siliconé lorsque l'amidon est un phosphate d'amidon,
lorsque la composition comprend au moins un agent épaississant et moins de 3% en poids de tensioactif, l'ester n'est pas le palmitate d'isopropyle ou l'adipate de diisopropyle.

2. Composition selon la revendication 1, **caractérisée par le fait que** les amidons sont choisis parmi les amidons de maïs, de riz, de manioc, de tapioca, d'orge, de pomme de terre, de blé, de sorgho, de pois.

3. Composition selon la revendication précédente, **caractérisée par le fait que** l'amidon est modifié par une ou plusieurs des réactions suivantes : prégélatinisation, oxydation, réticulation, estérification; traitements thermiques.

4. Composition selon l'une quelconque des revendications 1 à 5, **caractérisée par le fait que** l'ester est choisi parmi le béhénate d'octyldodécyle : le béhénate d'isocétyle ; le lactate d'isocétyle ; le lactate d'isostéaryle ; le lactate de linoléyle ; le lactate d'oléyle ; l'octanoate de isostéaryle ; l'octanoate d'isocétyle ; l'oléate de décyle ; l'isostéarate d'isocétyle ; le laurate d'isocétyle ; le stéarate d'isocétyle ; l'octanoate d'isodécyle ; l'oléate d'isodécyle ; l'isononanoate d'isononyle ; le palmitate d'isostéaryle : l'isostéarate de myristyle ; l'isononanoate d'octyle ; l'isononate de 2-éthylhexyle; l'isostéarate d'octyle ; l'érucate d'octyldodécyle ; les palmitates d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, l'isostéarate de butyle, le stéarate d'isobutyle ; le laurate de 2-hexyldécyle, le sébacate de diisopropyle ; l'adipate de diisopropyle ; l'adipate de diisostéaryle ; le stéarate d'octyldodécyl stéaroyl; le tétraisononanoate de pentaérythrityle ; le tétraisostéarate de pentaérythrityle ; le citrate de triisopropyle ; le citrate de triisotéaryle; le citrate de trioctyldodécyle.

5. Composition selon le revendication 6, **caractérisée par le fait que** l'ester est choisi parmi le palmitate d'isopropyle, le palmitate d'éthyl-2-héxyle, le palmitate de 2-octyldécyle, les myristates d'alkyles ramifiés tels que le myristate d'isopropyle, de t-butyle, de 2-octyldodécyle, l'isostéarate d'hexyle, le isostéarate de butyle, le stéarate d'isobutyle ; le laurate de 2-hexyldécyle et l'isononanate d'isononyle.

6. Composition selon rune quelconque des revendications 1 à 5, **caractérisée par le fait qu'**elle comprend en outre au moins un agent bénéfique pour les cheveux choisi parmi les huiles végétales, animales, minérales ou de synthèse, les cires, les céramides, les pseudocéramides, les silicones, les polymères cationiques, les filtres solaires, les vitamine.

7. Composition selon l'une quelconque des revendications précédentes, **caractérisée en ce que** l'amidon est présent à une concentration allant de 0,01 % à 20 % en poids par rapport au poids total de la composition, de préférence de 0,05 % à 15 % en poids.

8. Composition selon l'une quelconque des revendications précédentes, **caractérisée en ce que** l'ester carboxylique est présent à une concentration allant de 0,001 % à moins de 10 % en poids par rapport au poids total de la composition, de préférence de 0,01 % à 8 % en poids.

9. Composition selon l'une quelconque des revendications 6 à 8, **caractérisée en ce que** l'agent bénéfique pour les cheveux est présent à une concentration allant de 0,001 % à 20 % en poids par rapport au poids total de la composition, de préférence de 0,01 % à 10 % en poids.

10. Composition selon l'une quelconque des revendications précédentes, **caractérisée par le fait qu'**elle comprend en outre au moins un agent tensioactif choisi parmi les tensioactifs anioniques, non ioniques, amphotères, et leurs mélanges.

11. Composition selon l'une des revendications 1 à 10, **caractérisée par le fait que** le ou les agents tensioactifs sont présents à une concentration allant de 0,01% à 50% en poids, de préférence de 0,1% à 40% en poids, et encore plus préférentiellement de 0,5% à 30% en poids, par rapport au poids total de la composition.

12. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage.

13. Utilisation d'une, composition telle que définie dans l'une quelconque des revendications précédentes le lavage ou pour le soin des cheveux.

14. Utilisation d'une composition telle que définie dans l'une quelconque des revendications précédentes pour te traitement, le conditionnement des cheveux.

15. Utilisation d'une composition selon l'une quelconque des revendications 1 à 12, comme après-shampoing.

16. Procédé de traitement des cheveux, **caractérisé en ce qu'**il consiste à appliquer sur lesdits cheveux une composition cosmétique selon l'une des revendications 1 à 12, puis à effectuer éventuellement un rinçage à l'eau.

## Claims

1. Cosmetic hair composition, **characterized in that** it comprises in a cosmetically acceptable medium
a - at least one starch selected from monostarch phosphates, distarch phosphates, tristarch phosphates, and mixtures thereof, and from amphoteric starches,
b - at least one carboxylic ester selected from
liquid esters of C3-C30 carboxylic acid and C1-C30 alcohol, at least one of the acid or the alcohol being branched, and
c - at least one cationic surfactant,
with the provisos that
said composition comprises a fatty acid soap concentration less than or equal to 1.5% by weight relative to the total weight of the composition, the composition contains at least 30% by weight of water and less than 20% by weight of fatty phase, the composition contains less than 10% by weight of fatty esters,
the composition contains no nonsilicone cationic polymer when the starch is a starch phosphate, and when the composition comprises at least one thickener and less than 3% by weight of surfactant, the ester is not isopropyl palmitate or diisopropyl adipate.

2. Composition according to Claim 1, **characterized in that** the starches are selected from corn starch, rice starch, manioc starch, tapioca starch, barley starch, potato starch, wheat starch, sorghum starch, and pea starch.

3. Composition according to the preceding claim, **characterized in that** the starch is modified by one or more of the following reactions: pregelatinization, oxidation, crosslinking, esterification, thermal treatments.

4. Composition according to any one of Claims 1 to 3, **characterized in that** the ester is selected from octyldodecyl behenate; isocetyl behenate; isocetyl lactate; isostearyl lactate; linoleyl lactate; oleyl lactate; isostearyl octanoate; isocetyl octanoate; decyl oleate; isocetyl isostearate; isocetyl laurate; isocetyl stearate; isodecyl octanoate; isodecyl oleate; isononyl isononanoate; isostearyl palmitate; myristyl isostearate; octyl isononanoate; 2-ethylhexyl isononate; octyl isostearate; octyldodecyl erucate; isopropyl palmitates, 2-ethylhexyl palmitate, 2-octyldecyl palmitate, branched alkyl myristates such as isopropyl myristate, t-butyl myristate, 2-octyldodecyl myristate, hexyl isostearate, butyl isostearate, isobutyl stearate; 2-hexyldecyl laurate; diisopropyl sebacate; diisopropyl adipate; diisostearyl adipate; octyldodecyl stearoyl stearate; pentaerythrityl tetraisononanoate; pentaerythrityl tetraisostearate; triisopropyl citrate; triisostearyl citrate; and trioctyldodecyl citrate.

5. Composition according to Claim 4, **characterized in that** the ester is selected from isopropyl palmitate, 2-ethylhexyl palmitate, 2-octyldecyl palmitate, branched alkyl myristates such as isopropyl myristate, t-butyl myristate, and 2-octyldodecyl myristate, hexyl isostearate, butyl
isostearate, and isobutyl stearate; 2-hexyldecyl laurate, and isononyl isononanoate.

6. Composition according to any one of Claims 1 to 5, **characterized in that** it further comprises at least one hair benefit agent selected from vegetable oils, animal oils, mineral oils or synthetic oils, waxes, ceramides, pseudoceramides, silicones, cationic polymers, sunscreens, and vitamins.

7. Composition according to any one of the preceding claims, **characterized in that** the starch is present at a concentration ranging from 0.01% to 20% by weight relative to the total weight of the composition, preferably from 0.05% to 15% by weight.

8. Composition according to any one of the preceding claims, **characterized in that** the carboxylic ester is present at a concentration ranging from 0.001% to less than 10% by weight relative to the total weight of the composition, preferably from 0.01% to 8% by weight.

9. Composition according to any one of Claims 6 to 8, **characterized in that** the hair benefit agent is present at a concentration ranging from 0.001% to 20% by weight relative to the total weight of the composition, preferably from 0.01% to 10% by weight.

10. Composition according to any one of the preceding claims, **characterized in that** it further comprises at least one surfactant selected from anionic, nonionic and amphoteric surfactants and mixtures thereof.

11. Composition according to one of Claims 1 to 10, **characterized in that** the surfactant or surfactants are present at a concentration ranging from 0.01% to 50% by weight, preferably from 0.1% to 40% by weight, and more preferably still from 0.5% to 30% by weight, relative to the total weight of the composition.

12. Composition according to any one of the preceding claims, **characterized in that** it is in the form of a shampoo, a conditioner, a composition for perming, straightening, colouring or bleaching the hair, or a rinse-off composition to be applied between the two steps of a perming or hair-straightening operation.

13. Use of a composition as defined in any one of the preceding claims for the washing or care of the hair.

14. Use of a composition as defined in any one of the preceding claims for the treatment and conditioning of the hair.

15. Use of a composition according to any one of Claims 1 to 12 as a conditioner.

16. Process for treating the hair, **characterized in that** it consists in applying to said hair a cosmetic composition according to one of Claims 1 to 12, followed optionally by rinsing with water.

## Patentansprüche

1. Kosmetische Zusammensetzung für die Haarbehandlung,
**dadurch gekennzeichnet, dass** sie in einem kosmetisch akzeptablen Medium enthält:
a - mindestens eine Stärke, die unter den Monostärkephosphaten, Distärkephosphaten, Tristärkephosphaten und deren Gemischen und den amphoteren Stärken ausgewählt ist;
b - mindestens einen Carbonsäureester, der unter den flüssigen Estern einer Carbonsäure mit 3 bis 30 Kohlenstoffatomen und eines Alkohols mit 1 bis 30 Kohlenstoffatomen ausgewählt ist, wobei zumindest die Säure oder der Alkohol verzweigt vorliegt; und
c- mindestens einen kationischen grenzflächenaktiven Stoff;
mit der Maßgabe, dass:
die Zusammensetzung eine Konzentration an Carbonsäureseifen von kleiner oder gleich 1,5 Gew.-%, bezogen auf das Gesamtgewicht der Zusammensetzung, enthält;
die Zusammensetzung mindestens 30 Gew.-% Wasser und weniger als 20 Gew.-% Fettphase enthält;
die Zusammensetzung weniger als 10 Gew.-% Fettsäureester enthält;
die Zusammensetzung kein nicht siliconiertes kationisches Polymer enthält, wenn die Stärke ein Stärkephosphat ist;
der Ester nicht Isopropylpalmitat oder Diisopropyladipat ist, wenn die Zusammensetzung mindestens ein Verdickungsmittel und weniger als 3 Gew.-% grenzflächenaktiven Stoff enthält.

2. Zusammensetzung nach Anspruch 1, **dadurch gekennzeichnet, dass** die Stärke unter Maisstärke, Reisstärke, Maniokstärke,
Tapiocastärke, Gerstenstärke, Kartoffelstärke, Weizenstärke, Hirsenstärke und Erbsenstärke ausgewählt sind.

3. Zusammensetzung nach dem vorhergehenden Anspruch, **dadurch gekennzeichnet, dass** die Stärke durch eine oder mehrere der folgenden Reaktionen modifiziert ist:
Vorverkleisterung, Oxidation, Vernetzung, Veresterung, thermische Behandlungen.

4. Zusammensetzung nach einem der Ansprüche 1 bis 5, **dadurch gekennzeichnet, dass** der Ester unter Octyldodecylbehenat; Isocetylbehenat; Isocetyllactat; Isostearyllactat; Linoleyllactat; Oleyllactat; Isostearyloctanoat; Isocetyloctanoat; Decyloleat; Isocetylisostearat; Isocetyllaurat; Isocetylstearat; Isodecyloctanoat; Isodecyloleat; Isononylisononanoat; Isostearylpalmitat; Myristylisostearat; Octylisononanoat; 2-Ethylhexylisononat; Octylisostearat; Octyldodecylerucat; Isopropylpalmitat, 2-Ethyl-hexylpalmitat, 2-Octyldecylpalmitat, Alkylmyristaten mit verzweigten Alkylgruppen, wie Isopropylmyristat, tert-Butylmyristat, 2-Octyldodecylmyristat, Hexylisostearat, Butylisostearat, Isobutylstearat; 2-Hexyldecyllaurat, Diisopropylsebacat; Diisopropyladipat; Diisostearyladipat; Octyldodecylstearoylstearat; Pentaerythrityltetraisononanoat; Pentaerythrityltetraisostearat; Trüsopropylcitrat; Triisostearylcitrat; Trioctyldodecylcitrat ausgewählt ist.

5. Zusammensetzung nach Anspruch 6, **dadurch gekennzeichnet, dass** der Ester unter Isopropylpalmitat, 2-Ethyl-hexylpalmitat, 2-Octyldecylpalmitat, Alkylmyristaten mit verzweigten Alkylgruppen, wie Isopropylmyristat, *tert*-Butylmyristat, 2-Octyldodecylmyristat, Hexylisostearat, Butylisostearat, Isobutylstearat; 2-Hexyldecyllaurat und Isononylisononanoat ausgewählt ist.

6. Zusammensetzung nach einem der Ansprüche 1 bis 5, **dadurch gekennzeichnet, dass** sie ferner mindestens einen für die Haare vorteilhaften Stoff enthält, der unter den pflanzlichen Ölen, tierischen Ölen, Mineralölen oder synthetischen Ölen, Wachsen, Ceramiden, Pseudoceramiden, Siliconen, kationischen Polymeren, Sonnenschutzfiltern und Vitaminen ausgewählt ist.

7. Zusammensetzung nach einem der vorhergehenden Ansprüche, **dadurch gekennzeichnet, dass** die Stärke in einer Konzentration von 0,01 bis 20 Gew.-%, bezogen auf das Gesamtgewicht der Zusammensetzung, und vorzugsweise im Bereich von 0,05 bis 15 Gew.-% enthalten ist.

8. Zusammensetzung nach einem der vorhergehenden Ansprüche, **dadurch gekennzeichnet, dass** der Carbonsäureester in einer Konzentration im Bereich von 0,001 bis weniger als 10 Gew.-%, bezogen auf das Gesamtgewicht der Zusammensetzung, und vorzugsweise im Bereich von 0,01 bis 8 Gew.-% vorliegt.

9. Zusammensetzung nach einem der Ansprüche 6 bis 8, **dadurch gekennzeichnet, dass** der für die Haare vorteilhafte Stoff in einer Konzentration von 0,001 bis 20 Gew.-%, bezogen auf das Gesamtgewicht der Zusammensetzung, und vorzugsweise im Bereich von 0,01 bis 10 Gew.-% vorliegt.

10. Zusammensetzung nach einem der vorhergehenden Ansprüche, **dadurch gekennzeichnet, dass** sie ferner mindestens einen grenzflächenaktiven Stoff enthält, der unter den anionischen, nichtionischen, amphoteren grenzflächenaktive Stoffen und deren Gemischen ausgewählt ist.

11. Zusammensetzung nach einem der Ansprüche 1 bis 10, **dadurch gekennzeichnet, dass** der oder die grenzflächenaktive(n) Stoff(e) in einer Konzentration von 0,01 bis 50 Gew.-%, vorzugsweise 0,1 bis 40 Gew.-% und noch bevorzugter 0,5 bis 30 Gew.-%, bezogen auf das Gesamtgewicht der Zusammensetzung, vorliegen.

12. Zusammensetzung nach einem der vorhergehenden Ansprüche, **dadurch gekennzeichnet, dass** sie als Haarwaschmittel, Produkt, das nach der Haarwäsche angewandt wird, als Zusammensetzung für dauerhafte Verformungen, Entkräuselungen, Färbungen oder Entfärbungen der Haare oder als Zusammensetzung, die ausgespült wird und zwischen den beiden Schritten einer dauerhaften Verformung oder Entkräuselung aufgetragen wird, vorliegt.

13. Verwendung einer Zusammensetzung, wie sie in einem der vorhergehenden Ansprüche definiert ist, für die Reinigung oder für die Pflege der Haare.

14. Verwendung einer Zusammensetzung, wie sie in einem der vorhergehenden Ansprüche definiert ist, für die Behandlung, die Konditionierung der Haare.

15. Verwendung einer Zusammensetzung nach einem der Ansprüche 1 bis 12 als Produkt, das nach der Haarwäsche angewandt wird.

16. Verfahren zur Behandlung der Haare, **dadurch gekennzeichnet, dass** es darin besteht, eine kosmetische Zusammensetzung nach einem der Ansprüche 1 bis 12 auf die Haare aufzubringen und gegebenenfalls anschließend mit Wasser zu spülen.
